(19) **Europäisches Patentamt**

**European Patent Office**

**Office européen des brevets**

(11) **EP 1 541 133 B1**

(12) **EUROPEAN PATENT SPECIFICATION**

(45) Date of publication and mention
of the grant of the patent:
**10.08.2005 Bulletin 2005/32**

(51) Int Cl.⁷: **A61K 9/20**, A61K 47/02,
A61K 31/522

(21) Application number: **03028222.2**

(22) Date of filing: **09.12.2003**

(54) **PHARMACEUTICAL FORMULATION OF VALACICLOVIR**

PHARMAZEUTISCHE ZUBEREITUNG ENTHALTEND VALACICLOVIR

FORMULATION PHARMACEUTIQUE DE VALACICLOVIR

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(43) Date of publication of application:
**15.06.2005 Bulletin 2005/24**

(73) Proprietor: **Helm AG**
**20097 Hamburg (DE)**

(72) Inventors:
 • **Lieb, Sonja, Dr.**
 **20255 Hamburg (DE)**
 • **Löffler, Uwe, Dr.**
 **25436 Tornesch (DE)**

(74) Representative: **Teipel, Stephan, Dr. et al**
**Lederer & Keller**
**Patentanwälte**
**Prinzregentenstrasse 16**
**80538 München (DE)**

(56) References cited:
**WO-A-96/22082** WO-A-03/022209
**US-A1- 2003 152 622**

**Description**

This invention relates to a pharmaceutical formulation of valaciclovir.

The compound 9-[(2-hydroxyethoxy)methyl]guanine, otherwise known as acyclovir possesses potent antiviral activity and is widely used in the treatment and prophylaxis of viral infections in humans, particularly infections caused by the herpes group of viruses (see, for example, Schaeffer et al, Nature, 272, 583 - 585 (1978), UK Patent No. 1523865, US Patent No. 4,199,574). However, acyclovir is poorly absorbed from the gastrointestinal tract upon oral administration and this low bioavailability means that multiple high doses of oral drug may need to be administered, especially for the treatment of less sensitive viruses or infections in order to achieve and maintain effective anti-viral levels in the plasma.

The L-valine ester of acyclovir (2-[2-amino-1,6-dihydro-6-oxopurin-9-yl)methoxy]ethyl L-valinate (herein referred to as valaviclovir) has been shown to possess much improved bioavailability whilst retaining the anti-viral properties of acyclovir. A preferred form of this compound is its hydrochloride salt which is herein referred to as valaciclovir hydrochloride. Valaciclovir and its salts including the hydrochloride salt are disclosed in US Patent No. 4,957,924, European Patent No. 0308065 and Beauchamp et al, Antiviral Chemistry and Chemotherapy, 3(3), 157 - 164 (1992). Tablets of valaciclovir are also generally disclosed in the US Patent No. 4, 957, 924 and European Patent No. 0308065.

WO 96/22082 discloses valaciclovir tablets containing colloidal silicon dioxide. The colloidal silicon dioxide is added to provide a robust tablet formulation being capable of consistently providing tablets substantially free of cracks and having a hardness such that the tablet not only has an acceptable crushing force but also does not break during tumbling.

Colloidal silicon dioxide is also known as flow regulating agent (Fiedler, Lexikon der Hilfsstoffe, Editio Cantor Verlag, 5. Aufl., 2002; Schmidt, Christin, Wirk- und Hilfsstoffe für Rezeptur, Defektur und Großherstellung, Wissenschaftliche Verlagsgesellschaft mbH, Stuttgart, 1999).

Good flow is a prerequisite for a successful manufacture of for example tablets and powder-filled capsules on a production scale. The physical flow properties and densities in particular of tabletting powders are of great technical importance as tabletting machines usually are filled in volumetrically. The weight of the resulting tablet is thus depending on the dies of the tabletting machine and the densities of the related powders. If the powder is showing great differences in bulk and tapped densities great variations in the filling/dosing of the tabletting machines will occur (Bauer, Frömming, Führer: Pharmazeutische Technologie, 1993, Georg Thieme Verlag, Stuttgart). Thus, in the preparation of pharmaceutical formulations good physical flow properties and low differences in bulk and tapped densities of the compositions used for the preparation of the pharmaceutical formulations are desirable.

During development of pharmaceutical formulations containing valaciclovir, often difficulties in the physical flow properties of the composition are encountered.

It is therefore an object of the invention to provide a pharmaceutical formulation of valaciclovir or a pharmaceutically acceptable salt thereof which does not encounter the above problems. In particular the formulation should be preparable from a composition having good physical flow properties and low differences in bulk and tapped densities.

It has now been found that the above problems can be overcome by adding titanium dioxide to the composition in the preparation of the pharmaceutical formulation.

Thus, the present invention relates to a pharmaceutical formulation comprising valaciclovir or a pharmaceutically acceptable salt thereof in admixture with titanium dioxide.

The pharmaceutical formulation of the present invention is a solid, preferably oral composition, such as tablets, capsules, granules, pellets or sachets. Particularly preferred the pharmaceutical formulation is in the form of tablets.

The titanium dioxide is preferably present in the pharmaceutical formulation in an amount of 0.05 to 3 wt.%, more preferably in an amount of 0.5 to 1.5 wt.% of the total weight of the formulation.

It has been found that advantageous properties regarding density, compressibility and flowability of the ingredient mixture before tabletting and regarding hardness and disintegration of the resulting tablets can be obtained if a highly dispersed titanium dioxide is employed. Such highly dispersed titanium dioxide has for example an average primary particle size in the range of 16 to 26 nm, preferably of about 21 nm. The specific surface area (BET) of the titanium dioxide can typically be in the range of 30 to 70 $m^2/g$, preferably 50 $\pm$ 15 $m^2/g$. The tapped density of the titanium dioxide according to DIN ISO 787/XI, Aug. 1983, can be in the range of 120 to 140 g/l, preferably about 130 g/l. The titanium dioxide can be a hydrophilic fumed titanium dioxide.

A preferred titanium dioxide with the above physical properties is a hydrophilic fumed titanium dioxide sold by Degussa under the trademark AEROXIDE® $TiO_2$ P 25. A further titanium dioxide grade which may be used in the pharmaceutical formulation of the present invention is $TiO_2$ P 25 S. Two or more different grades of titanium dioxide may be used within one formulation.

The pharmaceutical formulation of the present invention may comprise any suitable amount of the active ingredient, valaciclovir or a pharmaceutically acceptable salt thereof. Advantageously the formulation contains a high proportion of valaciclovir, such as at least 50 wt.%, more preferably at least 75% wt.% of the total weight of the formulation.

Preferably the pharmaceutical formulation comprises a pharmaceutically acceptable salt of valaciclovir, in particular valaciclovir hydrochloride.

The pharmaceutical formulation of the present invention may further comprise one or more pharmaceutically acceptable excipients, such as fillers, binding agents, lubricants and disintegrating agents.

As fillers conventional fillers known to the person skilled in the art may be used. Suitable fillers are for example lactose, starch and various celluloses. The amount of the fillers present in the pharmaceutical formulation is not particularly limited and can be, for example, in the range of 0 to 30 wt.%, preferably 10 to 20 wt.% of the total weight of the formulation.

The binding agent serves, for example to bind the particles together and improve tablet hardness. Preferably the binding agent is present in an amount of 1 to 5 wt.%, more preferably at 2 to 4 wt.% of the total weight of the formulation. The binding agent can for example be methylcellulose or more preferably povidone. The grade of povidone is advantageously K30.

The binding agent such as povidone can be added dry to the drug and other pharmaceutically acceptable excipients and then a granulating solvent (such as water or an alcohol, in particular ethanol) can be added, but preferably it is dissolved in the granulating solvent before adding to the drug.

The lubricant is suitably present in an amount of 0.1 to 2 wt.%, preferably about 1 wt.% of the total weight of the formulation. Although lubricants such as talc or sodium lauryl sulfate are suitable, preferably the lubricant is a stearate, more preferably an alkali metal stearate, such as magnesium stearate.

Although valaciclovir is very soluble, especially in its salt form, it is preferable if a disintegrating agent is present in the pharmaceutical formulation, suitably in an amount of 0.1 to 20 wt.%, more preferably at about 0.5 to 7 wt.% of the total weight of the formulation. For example, crosscarmellose sodium may be used as disintegrating agent or any other suitable disintegrating agent known to the person skilled in the art.

The present invention also provides the use of titanium dioxide as flow regulating agent in the manufacture of a pharmaceutical formulation of valaciclovir or a pharmaceutically acceptable salt thereof.

A further aspect of the present invention provides a process of preparing a pharmaceutical formulation of valaciclovir or a pharmaceutically acceptable salt thereof, comprising admixing valaciclovir or a pharmaceutically acceptable salt thereof with titanium dioxide and optionally with further pharmaceutically acceptable excipients. Advantageously the valaciclovir or a pharmaceutically acceptable salt thereof is admixed with at least part of the pharmaceutically acceptable excipients, the mixture is granulated and the granulates are mixed with the titanium dioxide and, if applicable, the remainder of the pharmaceutically acceptable excipients. The obtained mixture can then be tabletted.

In a preferred embodiment of the process of the present invention granules are formed by mixing valaciclovir or a pharmaceutically acceptable salt thereof with the filler and a solution of the binding agent in a granulating solvent such as water or an alcohol (preferably ethanol) and granulating the obtained mixture to form granules; drying the granules; blending the dried granules with the lubricant, the disintegration enhancer and the titanium dioxide; and then compressing the blended mixture to form a tablet.

The tablets may optionally be coated or scored and may be formulated so as to provide slow or controlled release of the active ingredient therein.

The invention is now illustrated in the following examples which are not to be construed as being limiting.

Examples

Tablets of the following formulations 1 - 3 were prepared by granulating 1, 2 and 3 with a solution of 4 in 5. After drying 6, 7 and, where applicable, 8 were added. The final mixture was further processed to tablets with a final weight of 700,0 mg.

| *Formulation 1:* | |
|---|---|
| 1) Valaciclovir HCl | 556,24 mg |
| 2) Pregelatinized Starch | 70,0 mg |
| 3) Lactose monohydrate | 34,26 mg |
| 4) Povidone K30 | 21,00 mg |
| 5) Alcohol | 378,0 mg |
| 6) Crosscarmellose Sodium | 4.50 mg |
| 7) Magnesium Stearate | 7.00 mg |
| 8) TiO$_2$ P 25 / TiO$_2$ P 25 S | 7.00 mg |

| Formulation 2: | |
|---|---|
| 1) Valaciclovir HCl | 556,24 mg |
| 2) Pregelatinized Starch | 70,0 mg |
| 3) Lactose monohydrate | 41,26 mg |
| 4) Povidone K30 | 21,00 mg |
| 5) Alcohol | 378,0 mg |
| 6) Crosscarmellose Sodium | 4.50 mg |
| 7) Magnesium Stearate | 7.00 mg |

| Formulation 3: | |
|---|---|
| 1) Valaciclovir HCl | 556,24 mg |
| 2) Pregelatinized Starch | 70,0 mg |
| 3) Lactose monohydrate | 34,26 mg |
| 4) Povidone K30 | 21,00 mg |
| 5) Alcohol | 378,0 mg |
| 6) Crosscarmellose Sodium | 4.50 mg |
| 7) Magnesium Stearate | 7.00 mg |
| 8) $SiO_2$ | 7.00 mg |

The following physical parameters of the final mixtures and the obtained tablets were measured:

Hausner Factor

The Hausner Factor is calculated by the following formula:

$$\frac{\text{bulk density}}{\text{tapped density}}$$

The bulk density is measured according to Ph Eur 2.9.15 (European Pharmacopoeia, 4. Ed., published by the Directorate for the Quality of Medicines of the Council of Europe) as poured density. The tapped density is measured according to Ph Eur 2.9.15.
The Hausner Factor is a measure for the flowability/compressibility of powders and should be around 1. Preferably mixtures for preparing tablets should have a Hausner Factor of <1.16.

Compressibility Index

The compressibility index is calculated by the formula:

$$\frac{100\ (V_0 - V_{300})}{V_0}$$

wherein $V_0$ is the bulk volume and $V_{300}$ is the tapped volume of the mixture, both measured as described in W. A. Ritschel and A. Bauer-Brandl, Die Tablette, p. 355f, 2. Ed., Editio Cantor Verlag, Aulendorf, 2002.
A compressibility below 15% indicates a good flow, above 25% a difficult flow.

Flowability

The flowability is measured according to Ph Eur 2.9.16.

Pile angle

Approximately 50 g sample is taken and the flowability test is applied as described above. The sample that comes out from the funnel is collected over a clean paper without changing the conical shape of the collected sample. Firstly the surroundings of this cone is drawn on the paper to determine the diameter. Secondly the height (=h) of this cone is measured by means of a thin pipe. The diameter of the circle on the paper is measured and recorded (=D). To obtain the radius of the circle the diameter is divided by two (D/2=r).

After obtaining the r and h values the following equation is used to calculate the pile angle:

$$\text{pile angle (Tan } \alpha) = \frac{\text{Height (h)}}{\text{Radius (r)}}$$

A pile angle $\alpha \leq 30°$ indicates good flow, a pile angle of $30° < \alpha \leq 40°$ indicates a difficult flow and a pile angle of $\alpha > 40°$ indicates a very bad flow.

Hardness

Tablets should have a high hardness to avoid break during tumbling. The hardness is measured according to Ph Eur 2.9.8.

Disintegration

The time required for disintegration of the tablets is measured according to Ph Eur 2.9.1. For immediate release tablets the time should be <15 min.

The results of the measurements are summarized in the following table:

|  | Formulation 1 | Formulation 2 | Formulation 3 |
|---|---|---|---|
| Hausner Factor | 1.14 | 1.41 | 1.31 |
| Compressibility Index | 12,61% | 25,61% | 23,8% |
| Flowability | 4 sec. | 19 sec. | 15 sec. |
| Pile angle ($\alpha$) | 30.1° | 40,5° | 34,2° |
| Hardness | 180 N | 90 N | 130 N |
| Disintegration | 12'15" - 14'10" | 17'15" - 22'10" | 16'20" - 18'20" |

The above results show that by adding titanium dioxide a tablet mass (formulation 1) with very good flow properties and thus very good tabletting behaviour is obtained. The tablets prepared from this mass have a high hardness and at the same time a short disintegration time. On contrary, as shown with formulations 2 and 3, the "classic" flow regulating agent $SiO_2$ (formulation 3) does not give comparably good results, although they are still better than without adding any flow regulating agent (formulation 2).

**Claims**

1.   Pharmaceutical formulation comprising valaciclovir or a pharmaceutically acceptable salt thereof in admixture with titanium dioxide.

2.   Pharmaceutical formulation according to claim 1, said formulation being in the form of tablets, capsules, granules, pellets or sachets.

3.   Pharmaceutical formulation according to any of the proceeding claims, wherein the titanium dioxide is present in an amount of 0.05 to 3 wt.% of the total weight of the formulation.

4.   Pharmaceutical formulation according to claim 3, wherein the titanium dioxide is present in an amount of 0.5 to 1.5 wt.% of the total weight of the formulation.

5.   Pharmaceutical formulation according to any of the proceeding claims, wherein the titanium dioxide is a highly dispersed titanium dioxide.

6. Pharmaceutical formulation according to claim 5, wherein the titanium dioxide has an average primary particle size in the range of 16 to 26 nm, preferably of about 21 nm.

7. Pharmaceutical formulation according to any of the proceeding claims, wherein the valaciclovir or a pharmaceutically acceptable salt thereof is present in an amount of at least 50 wt.% of the total weight of the formulation.

8. Pharmaceutical formulation according to any of the proceeding claims, further comprising one or more pharmaceutically acceptable excipients, such as fillers, binding agents, lubricants and disintegrating agents.

9. Use of titanium dioxide as flow regulating agent in the manufacture of a pharmaceutical formulation of valaciclovir or a pharmaceutically acceptable salt thereof.

10. Process of preparing a pharmaceutical formulation of valaciclovir or a pharmaceutically acceptable salt thereof, comprising admixing valaciclovir or a pharmaceutically acceptable salt thereof with titanium dioxide and optionally with further pharmaceutically acceptable excipients.

11. Process according to claim 10 wherein the valaciclovir or a pharmaceutically acceptable salt thereof is admixed with at least part of the pharmaceutically acceptable excipients, the mixture is granulated and the granulates are mixed with the titanium dioxide and, if applicable, the remainder of the pharmaceutically acceptable excipients.

12. Process according to claim 11, further comprising the step of tabletting the obtained mixture.


**Patentansprüche**

1. Pharmazeutische Formulierung, umfassend Valaciclovir oder ein pharmazeutisch akzeptables Salz hiervon, in Beimischung mit Titaniumdioxid.

2. Pharmazeutische Formulierung nach Anspruch 1, wobei die Formulierung in Form von Tabletten, Kapseln, Granulaten, Pellets oder Beuteln vorliegt.

3. Pharmazeutische Formulierung nach einem der vorhergehenden Ansprüche, wobei das Titaniumdioxid in einer Menge von 0,05 bis 3 Gew.-% des Gesamtgewichts der Formulierung vorliegt.

4. Pharmazeutische Formulierung nach Anspruch 3, wobei das Titaniumdioxid in einer Menge von 0,5 bis 1,5 Gew.-% des Gesamtgewichts der Formulierung vorliegt.

5. Pharmazeutische Formulierung nach einem der vorhergehenden Ansprüche, wobei das Titaniumdioxid hochdisperses Titaniumdioxid ist.

6. Pharmazeutische Formulierung nach Anspruch 5, wobei das Titaniumdioxid eine durchschnittliche Primärteilchengröße im Bereich von 16 bis 26 nm, bevorzugt von etwa 21 nm, aufweist.

7. Pharmazeutische Formulierung nach einem der vorhergehenden Ansprüche, wobei das Valaciclovir oder ein pharmazeutisch akzeptables Salz hiervon in einer Menge von zumindest 50 Gew.-% des Gesamtgewichts der Formulierung vorliegt.

8. Pharmazeutische Formulierung nach einem der vorhergehenden Ansprüche, die ferner einen oder mehrere pharmazeutisch akzeptable Trägerstoffe, wie Füllstoffe, Bindemittel, Schmiermittel und Desintegrationsmittel, umfaßt.

9. Verwendung von Titaniumdioxid als Strömungsregulierungsmittel bei der Herstellung einer pharmazeutischen Formulierung aus Valaciclovir oder einem pharmazeutisch akzeptablen Salz hiervon.

10. Verfahren zur Herstellung einer pharmazeutischen Formulierung aus Valaciclovir oder einem pharmazeutisch akzeptablen Salz hiervon, umfassend die Beimischung von Valaciclovir oder einem pharmazeutisch akzeptablen Salz hiervon, mit Titaniumdioxid und gegebenenfalls mit weiteren pharmazeutisch akzeptablen Trägerstoffen.

11. Verfahren nach Anspruch 10, wobei das Valaciclovir oder ein pharmazeutisch akzeptables Salz hiervon mit zumindest einem Teil der pharmazeutisch akzeptablen Trägerstoffe gemischt wird, das Gemisch granuliert wird, und die Granulate mit dem Titaniumdioxid und, wenn zutreffend, dem Rest der pharmazeutisch akzeptablen Trägerstoffe, gemischt werden.

**12.** Verfahren nach Anspruch 11, ferner umfassend den Schritt der Tablettierung des erhaltenen Gemisches.

**Revendications**

**1.** Formulation pharmaceutique comprenant du valaciclovir ou un de ses sels acceptables sur le plan pharmaceutique en mélange avec du dioxyde de titane.

**2.** Formulation pharmaceutique selon la revendication 1, ladite formulation étant sous la forme de comprimés, de gélules, de granulés, de pilules ou de sachets.

**3.** Formulation pharmaceutique selon l'une quelconque des revendications précédentes, dans laquelle le dioxyde de titane est présent à raison de 0,05% à 3% en poids par rapport au poids total de la formulation.

**4.** Formulation pharmaceutique selon la revendication 3, dans laquelle le dioxyde de titane est présent à raison de 0,5% à 1,5% en poids par rapport au poids total de la formulation.

**5.** Formulation pharmaceutique selon l'une quelconque des revendications précédentes, dans laquelle le dioxyde de titane est un dioxyde de titane fortement dispersé.

**6.** Formulation pharmaceutique selon la revendication 5, dans laquelle le dioxyde de titane possède une taille de particules primaires moyenne dans la gamme de 16 nm à 26 nm, de préférence d'environ 21 nm.

**7.** Formulation pharmaceutique selon l'une quelconque des revendications précédentes, dans laquelle le valaciclovir ou un de ses sels acceptables sur le plan pharmaceutique est présent à raison d'au moins 50% en poids par rapport au poids total de la formulation.

**8.** Formulation pharmaceutique selon l'une quelconque des revendications précédentes, comprenant en outre un ou plusieurs excipients acceptables sur le plan pharmaceutique, tels que des charges, des agents de liaison, des lubrifiants et des agents de délitement.

**9.** Utilisation de dioxyde de titane en tant qu'agent de régulation de l'écoulement dans la préparation d'une formulation pharmaceutique de valaciclovir ou d'un de ses sels acceptables sur le plan pharmaceutique.

**10.** Procédé de préparation d'une formulation pharmaceutique de valaciclovir ou d'un de ses sels acceptables sur le plan pharmaceutique, consistant à mélanger le valaciclovir ou un de ses sels acceptables sur le plan pharmaceutique avec du dioxyde de titane et éventuellement avec d'autres excipients acceptables sur le plan pharmaceutique.

**11.** Procédé selon la revendication 10, dans lequel le valaciclovir ou un de ses sels acceptables sur le plan pharmaceutique est mélangé avec au moins une partie des excipients acceptables sur le plan pharmaceutique, le mélange est granulé et les granulés sont mélangés avec le dioxyde de titane et, le cas échéant, le reste des excipients acceptables sur le plan pharmaceutique.

**12.** Procédé selon la revendication 11, comprenant en outre l'étape consistant à mettre le mélange obtenu sous forme de comprimés.